Europäisches Patentamt

European Patent Office

Office européen des brevets

(19)

(11) Veröffentlichungsnummer : **0 125 713**
**B1**

(12)

# EUROPÄISCHE PATENTSCHRIFT

(45) Veröffentlichungstag der Patentschrift :
28.09.88

(51) Int. Cl.⁴ : **A 61 B   6/04**

(21) Anmeldenummer : **84200519.1**

(22) Anmeldetag : **12.04.84**

(54) **Um eine horizontale Achse schwenkbares Röntgengerät.**

(30) Priorität : **18.04.83 DE 3313994**

(43) Veröffentlichungstag der Anmeldung :
**21.11.84 Patentblatt 84/47**

(45) Bekanntmachung des Hinweises auf die Patenterteilung : **28.09.88 Patentblatt 88/39**

(84) Benannte Vertragsstaaten :
**BE DE FR GB IT**

(56) Entgegenhaltungen :
**DE-C-   970 321**
**US-A- 2 692 173**

(73) Patentinhaber : **Philips Patentverwaltung GmbH**
**Wendenstrasse 35 Postfach 10 51 49**
**D-2000 Hamburg 1 (DE)**
**DE**
**N.V. Philips' Gloeilampenfabrieken**
**Groenewoudseweg 1**
**NL-5621 BA Eindhoven (NL)**
**BE FR GB IT**

(72) Erfinder : **Schmedemann, Walter**
**Dorfstrasse 80**
**D-2000 Tangstedt (DE)**

(74) Vertreter : **Hartmann, Heinrich, Dipl.-Ing. et al**
**Philips Patentverwaltung GmbH Wendenstrasse 35**
**Postfach 10 51 49**
**D-2000 Hamburg 1 (DE)**

EP 0 125 713 B1

**Beschreibung**

Die Erfindung betrifft ein Röntgengerät nach dem Oberbegriff des Hauptanspruchs. Ein derartiges Gerät ist aus der DE-PS 970 321 bekannt. Dieses Gerät ist in eine Stellung schwenkbar, in der die Tischplatte senkrecht steht (Fußtieflage) und ist aus dieser Stellung über die Horizontalstellung der Tischplatte hinaus in eine sogenannte Kopftieflage schwenkbar. Da die Schwenkachse dabei relativ niedrig liegen muß, ist es erforderlich, während der Schwenkbewegung den Rahmen innerhalb des Führungsteils in Tischlängsrichtung so zu verschieben, daß er in den beiden äußeren Schwenkstellungen nicht mit dem Fußboden kollidiert.

Diesem Zweck dient bei dem bekannten Gerät die mit der Hebelanordnung gekoppelte Schubstange, wodurch die Schwenkbewegung des Führungsteils eine Längsverschiebung des Tischrahmens hervorruft. Die Hebelanordnung ·bei dem bekannten Gerät ist allerdings nur wirksam, wenn der Rahmen über die Horizontallage hinaus in die Kopftieflage geschwenkt wird, wobei ein Nocken, der am Führungsteil befestigt ist, auf die Hebelanordnung einwirkt. Das Aktivieren der Hebelanordnung durch den Nocken erfolgt stoßartig, wodurch sich Erschütterungen des Gerätes ergeben. Wenn das Gerät über die Horizontallage hinaus in Richtung auf das Fußende geschwenkt und der Nocken nicht mehr auf die Hebelanordnung einwirkt, kann der Rahmen in Richtung auf. das Kopfende frei verschoben werden, d. h. seine Position ist dann unabhängig von der Schwenkstellung des Gerätes.

Aufgabe der vorliegenden Erfindung ist es, ein Röntgengerät der eingangs genannten Art so auszugestalten, daß es keinen stoßartigen Übergang mehr gibt und daß die Position des Rahmens eindeutig durch die Schwenkstellung des Führungsteils definiert ist. Diese Aufgabe wird erfindungsgemäß durch die im Kennzeichen des Hauptanspruchs angegebenen Maßnahmen gelöst.

Das stabartige Glied, der damit verbundene Hebelarm und die (durch das Führungsteil vorgegebene) starre Verbindung zwischen dem Lagerpunkt und der Schwenkachse bilden dabei ein Gelenkviereck, das durch die Schwenkung des Führungsteils angetrieben wird, wobei sich der erwähnte Hebelarm um den Lagerpunkt des Hebels dreht. Diese Drehbewegung wird auf den anderen Hebelarm und die Schubstange — diese beiden Teile bilden zusammen eine sogenannte Schubkurbel — übertragen, wodurch der Rahmen innerhalb des Führungsteils verschoben wird.

Da hierbei alle Teile (Rahmen, Führungsteil, Fußgestell) über starre Kraftübertragungsglieder miteinander verbunden sind, ist in jeder Schwenkstellung des Gerätes gewährleistet, daß der Rahmen relativ zum Führungsteil eine definierte Position einnimmt. Da alle Kraftübertragungsglieder ständig wirksam sind, kann der stoßartige Übergang, der bei dem bekannten Gerät beim Eingreifen des Nockens in die Hebelanordnung auftritt, vermieden werden.

Die Erfindung wird nachstehend anhand von Zeichnungen näher erläutert. Es zeigen

Fig. 1 eine Frontansicht des Gerätes (vom Fußende aus) bei horizontaler Lage des Rahmens,

Fig. 2a bis 2c eine schematische Seitenansicht in verschiedenen Schwenkstellungen, wobei die für das Verständnis der Erfindung nicht notwendigen Teile weggelassen sind.

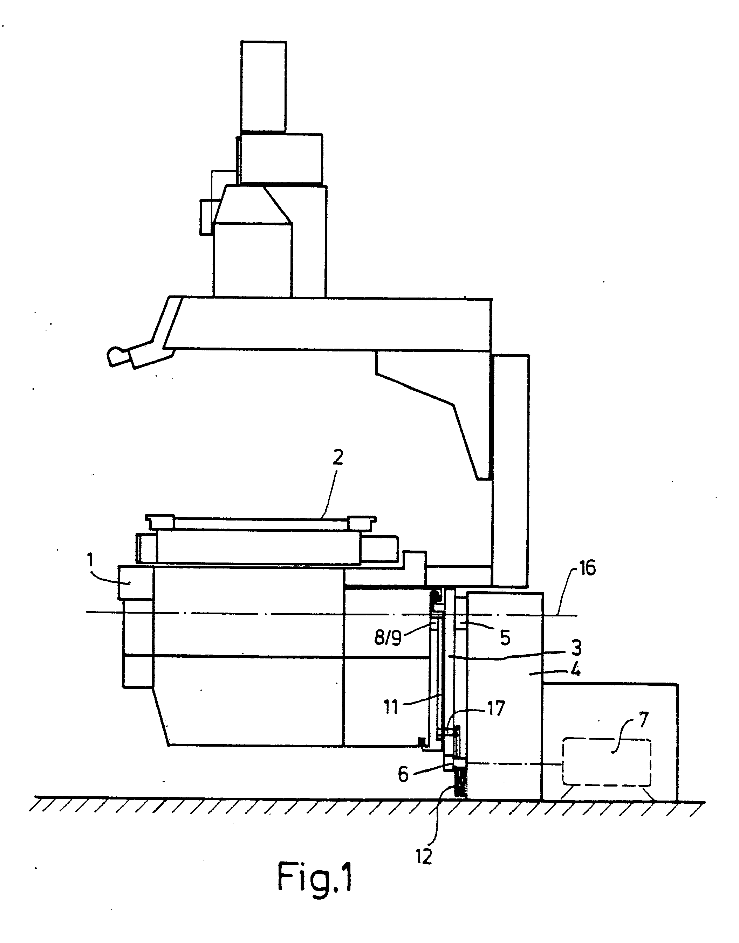

Das in Fig. 1 dargestellte Röntgengerät umfaßt einen Rahmen 1, der eine Tischplatte 2 trägt und der in einem Führungsteil 3 in Längsrichtung der Tischplatte verschiebbar ist (d. h. senkrecht zur Zeichenebene der Fig. 1) und von diesem getragen wird. Der Führungsteil 3 ist mit einem Fußgestell 4, auf dem das gesamte Gerätegewicht ruht, über ein Lager 5 verbunden, so daß der Führungsteil um die zur Tischlängsrichtung senkrechte horizontale Achse 16 schwenkbar ist, die durch das Zentrum dieses Lagers verläuft.

Der Führungsteil 3 ist als massives halbkreisförmiges Segment ausgebildet, dessen Krümmungsmittelpunkt mit der Schwenkachse 16 zusammenfällt und das auf seinem äußeren Umfang mit einer Verzahnung versehen ist, in die ein Antriebsritzel 6 eingreift, auf das ein Antriebsmotor 7 einwirkt.

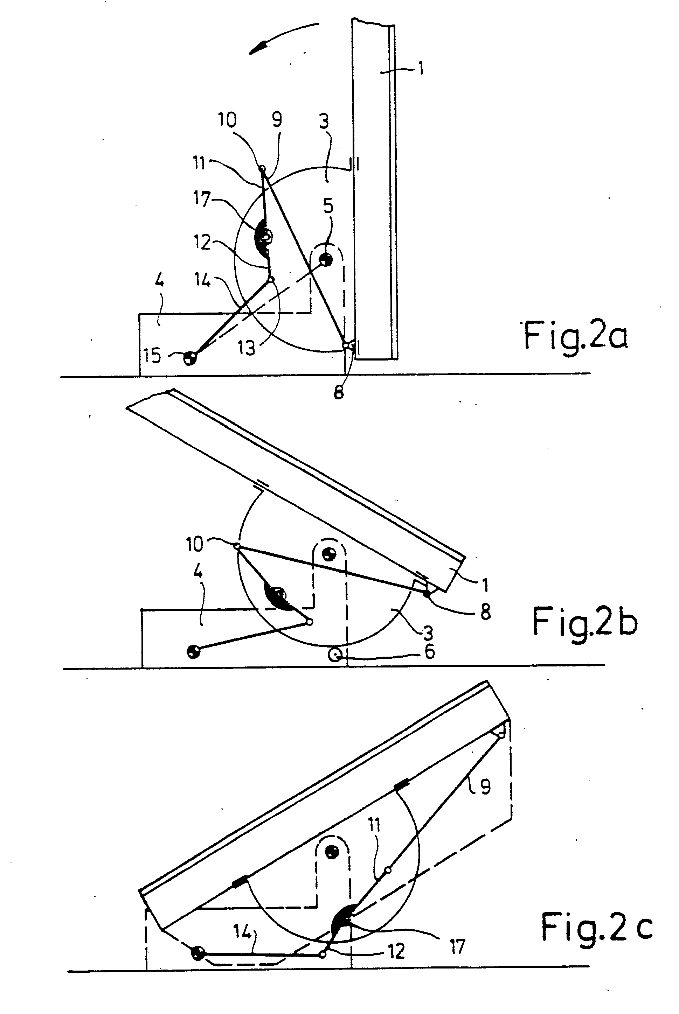

Wie sich insbesondere aus den Fig. 2a bis 2c ergibt, die das erfindungsgemäße Röntgengerät in einer vertikalen Tischplattenstellung (Fußtieflage — Fig. 2a), in einer leicht zum Fußende hin geneigten Lage (Fig. 2b) und in der maximal erreichbaren Kopftieflage (Fig. 2c) gezeigt, ist am Fußende des Rahmens 1 bei 8 eine Schubstange 9 angelenkt, die bei 10 mit dem einen Arm 11 eines zweiarmigen Hebels verbunden ist, dessen anderer Arm 12 bei 13 gelenkig mit einem Stab 14 verbunden ist, der seinerseits um ein Drehgelenk 15 am Fußgestell 4 schwenkbar ist. Der zweiarmige Hebel d. h. die Verbindungsstelle der beiden Hebelarme 11 und 12 ist im Abstand von dem Lager 5 bei 17 im Führungsteil gelagert, so daß das Lager 17 bei einer Schwenkung des Gerätes sich auf einem Kreisbogen um das Lager 5 bzw. die horizontale Schwenkachse bewegt.

Der Stab 14, der Hebelarm 12 und die durch das Führungsteil 3 gegebene starre Verbindung zwischen den Lagern 5 und 17 bilden ein Gelenkviereck mit den Drehgelenken 5, 15, 13 und 17 (von denen 5 und 15 ortsfest sind), wobei die Verbindung 5 (16) — 17 das angetriebene Glied — die sogenannte Kurbel — bildet, während die über den Hebelarm 12 — dieses Glied wird im Gelenkviereck als « Koppel » bezeichnet — den Stab 14 — die sogenannte « Schwinge » um das Lager 15 schwenkt. Die damit einhergehende Schwenkung des Hebelarmes 12 um das Lager 16 wird auf eine sogenannte Schubkurbel übertragen, die durch den Hebelarm 11 und die Schubstange 9 gebildet wird, wobei der Hebelarm 11 das antreibende Glied (das seinerseits von dem Hebelarm 12 angetrieben wird) darstellt — die sogenannte Kurbel — und die Schubstange 9 das angetriebene Glied bildet, die ihrerseits den Rahmen 1 geradlinig relativ zum Führungsteil 3 verschiebt.

Wie sich aus dem vorherstehenden ergibt, wird der Rahmen 1 nur dann verschoben, wenn der zweiarmige Hebel um sein Lager 17 gedreht wird. Eine solche Drehung würde nicht erfolgen, wenn bei einer Schwenkbewegung des Röntgengerätes das Gelenk 13 zwischen dem Stab 14 und dem Hebelarm 12 auf einem Kreis um das Lager 5 bzw. die Schwenkachse 16 (Fig. 1) umlaufen würde. Wenn die Bahn des Gelenkes 13 nur wenig von einer solchen Kreisbahn abweicht, bleibt auch die Drehung des Hebels um das Lager 17 gering — und mithin auch die Verschiebung des Rahmens 1 innerhalb des segmentförmigen Führungsteils 3.

Wie sich aus Fig. 2a ergibt, ist in der Vertikalstellung des Gerätes der Winkel zwischen dem Stab 14 und der Verbindungsgeraden zwischen den Punkten 5 und 15 relativ gering. Infolgedessen bewegt sich bei einer Schwenkung des Gerätes aus dieser Stellung in Richtung auf die Horizontallage das Gelenk 13 auf einem Kreisbogen um das Lager 15, der annähernd mit einem Kreisbogen um das Lager 5 zusammenfällt, so daß bei einer Schwenkung aus der in Fig. 2a dargestellten Fußtieflage zunächst der Hebel 12 kaum gedreht und der Rahmen 1 kaum verschoben wird. Es kommt hinzu, daß in dieser Gerätestellung der Winkel zwischen dem Hebelarm 11 und der damit verbundenen Schubstange 9 relativ spitz ist, so daß eine Drehung des Hebels 11, 12 um das Lager 17 sich ohnehin im Hinblick auf die Verschiebung des Rahmens 1 nur wenig bemerkbar macht.

Dies alles führt dazu daß bei der Schwenkung des Gerätes aus der Stellung nach Fig. 2a in die Stellung nach Fig. 2b der Rahmen 1 kaum in Richtung auf das Fußende verschoben wird, so daß er infolge der Verschiebung auch nicht mit dem Fußboden kollidieren kann.

Bei der weiteren Schwenkung des Tisches (aus der Stellung nach Fig. 2b) im Gegenuhrzeigersinn ändern sich die Verhältnisse jedoch wesentlich: Im Bereich der horizontalen Stellung des Tischrahmens bewegt sich das Gelenk 13 nämlich auf einem Kreisbogen, der nahezu senkrecht zu einem Kreis um das Lager 5 verläuft, so daß der Hebel 11, 12 relativ stark um das Lager 17 gedreht wird, und gleichzeitig beträgt der Winkel zwischen dem Hebelarm 11 und der Schubstange 9 annähernd 90°. Beides führt dazu, daß im Bereich der Horizontalstellung des Gerätes eine relativ starke Verschiebung des Rahmens (bezogen auf die Schwenkung des Gerätes) erfolgt, was zur Folge hat, daß der überwiegende Teil der Verschiebung im Bereich der Horizontalstellung erfolgt. Dies ist von Vorteil, weil in dieser Stellung des Gerätes das Gewicht des Tischrahmens sich nur geringfügig auf die zur Verschiebung erforderlichen Kräfte auswirkt.

Fig. 2c zeigt das Gerät in seiner extremen Kopftieflage, wobei zur Verdeutlichung die Umrisse der Verkleidung des Tischrahmens gestrichelt angedeutet werden, wobei durch den Hebel 11 und die Schubstange 9 gebildete Schubkurbel vollständig gestreckt ist. Wie aus den Fig. 2a bis 2c zu erkennen, ist der Hebel 11, 12 leicht abgewinkelt, d. h. der Hebel 11 schließt mit dem Hebel 12 (im Uhrzeigersinn gemessen) einen Winkel von weniger als 180° ein. Durch diese leichte Abwinkelung wird verhindert, daß der Arm 11 bei aufrechtem Gerät über die Gerätekontur hinausragt.

Wie insbesondere aus Fig. 1 hervorgeht, sind der Rahmen 1 und das Fußgestell 4 auf verschiedenen Seiten des segmentförmigen Führungsteils 3 angeordnet. Da nun die Schubstange 9 am Rahmen, der Stab 14 jedoch am Fußgestell angreifen muß, können die Schubstange 9, die Hebelarme 11 und 12 und der Stab 14 nicht alle in der gleichen Ebene geführt werden. Deshalb ist der Hebelarm 11 und die Schubstange 9 zwischen dem Führungsteil 3 und dem Rahmen 1 angeordnet, während der Hebelarm 12 und der Stab 14 zwischen dem Führungsteil 3 und dem Fußgestell 4 angeordnet sind.

Die Hebelarme 11 und 12 sind über das Lager 17 durch das Führungsteil 3 hindurch starr miteinander verbunden. Diese Anordnung hat den Vorteil, daß der Hebelarm 11 so lang sein kann, daß er — wäre er auf der anderen Seite des Führungsteils 3 — mit dem Lager 5 kollidieren würde; allerdings setzt dies voraus, daß in der vertikalen Stellung des Hebelarmes 11 die Führung des Rahmens in dem Führungsteil oberhalb des Gelenkes 10 verläuft.

Ein praktisch erprobtes Gerät hatte folgende Abmessungen:

    Schubstange 9 — 777 mm
    Hebelarm 11 — 305 mm
    Hebelarm 12 — 200 mm
    Stab 14 — 563 mm

Das Lager 5 hatte von dem 79 mm oberhalb des Bodens befindlichen Gelenk 15 einen Abstand von 813 mm und befand sich 553 mm oberhalb des Bodens.

Der Abstand des Lagers 17 vom Lager 5 betrug 326 mm, wobei der Winkel zwischen der Verbindungslinie 17-5 67° betrug. Der Winkel zwischen den Hebelarmen 11 und 12 betrug 172°, und die zum Tischrahmen parallele Ebene, in der das Gelenk 8 geführt ist, verlief in einem Abstand von 9,5 mm. Mit dieser Auslegung konnte eine Kopftieflage von 30° bei einem Hub des Tischrahmens bei der Schwenkbewegung von 640 mm erreicht werden.

Mit den gleichen Bauteilen konnte der gleiche Hub bei einm Gerät mit nur 15° Kopftieflage erreicht werden, wobei lediglich der Punkt, an dem das Gelenk 15 mit dem Fußgestell 4 verbunden ist, versetzt werden mußte.

**Patentansprüche**

1. Röntgengerät mit einem eine Tischplatte (2) tragenden Rahmen (1), der in Längsrichtung verschiebbar von einem Führungsteil (3) getragen

**0 125 713**

wird, der in einem Fußgestell (4) um eine horizontale Schwenkachse (16) schwenkbar gelagert ist, wobei eine Schwenkbewegung des Führungsteils (3) mittels einer am Rahmen (1) angelenkten Schubstange (9) in eine Längsverschiebung umsetzbar ist, dadurch gekennzeichnet, daß eine Hebelanordnung einen zweiarmigen Hebel (11, 12) umfaßt, der um ein Drehgelenk (17) schwenkbar im Abstand von der Schwenkachse (16) am Führungsteil (3) gelagert ist, daß ein stabartiges Glied (14) am Fußgestell (4) um eine zur Schwenkachse (16) parallele Achse (15) schwenkbar angeordnet ist, und daß die freien Enden (13, 10) des stabartigen Gliedes (14) und der Schubstange (9) mit den beiden Hebelarmen (11, 12) gelenkig verbunden sind.

2. Röntgengerät nach Anspruch 1, dadurch gekennzeichnet, daß der mit der Schubstange (9) verbundene Hebelarm (11) auf der vom Fußgestell (4) abgewandten Seite des Führungsteils (3) angeordnet ist, daß der mit dem stabartigen Glied (14) verbundene Hebelarm (12) auf der anderen Seite des Führungsteils (3) angeordnet ist, und daß die beiden Hebelarme (11, 12) durch das Führungsteil (3) hindurch starr miteinander verbunden sind.

3. Röntgengerät nach einem der vorhergehenden Ansprüche, gekennzeichnet durch eine solche Ausbildung, daß im Bereich der vertikalen Stellung (Fußtieflage) des Gerätes die Gelenkverbindung (13) zwischen dem stabartigen Glied (14) und dem einen Hebelarm (12) einen Kreisbogen beschreibt, der die Verbindungsgerade zwischen der Schwenkachse (16) und dem Punkt schneidet, an dem das stabartige Glied (14) am Fußgestell (4) angelenkt ist.

4. Schaltungsanordnung nach einem der vorhergehenden Ansprüche, dadurch gekennzeichnet, daß in der Fußtieflage der Winkel zwischen der Schubstange (9) und dem damit verbundenen Hebelarm (11) möglichst spitz ist.

**Claims**

1. An X-ray apparatus, comprising a frame (1) which supports a tabletop (2) and which is supported so as to be movable in a longitudinal direction by a guide (3) which is mounted in a pedestal (4) so as to be pivotable about a horizontal pivot axis (16), a tilting motion of the guide (3) being convertible into a longitudinal motion by means of a connecting rod (9) linked to the frame (1), characterized in that a lever arrangement comprises a two-arm lever (11, 12) which is mounted on the guide (3) so as to be pivotable about a pivot joint (17) at a distance from the pivot axis (16), a rod-like element (14) being mounted on the pedestal (4) so as to pivot about an axis (15) parallel to the pivot axis (16), the free ends (13, 10) of the rod-like element (14) and the connecting rod (9) being articulated with the two lever arms (11, 12).

2. An X-ray apparatus as claimed in Claim 1, characterized in that the lever arm (11) joined to the connecting rod (9) is arranged at the side of the guide (3) which is remote from the pedestal (4), the lever arm (12) joined to the rod-like element (14) being arranged at the other side of the guide (3), the two lever arms (11, 12) being rigidly interconnected through the guide (3).

3. An X-ray apparatus as claimed in any one of the preceding Claims, characterized in that it is constructed so that at the area of the vertical position (low foot position) of the apparatus unit the linkage (13) between the rod-like element (14) and one lever arm (12) describes an arc of circle which intersects the straight line connecting the pivot axis (16) and the point at which the rod-like element (14) is articulated with the pedestal (4).

4. A circuit arrangement as claimed in any one of the preceding Claims, characterized in that in the low foot position the angle between the connecting rod (9) and the lever arm (11) connected thereto is as acute as possible.

**Revendications**

1. Appareil à rayons X comportant un cadre (1) portant un plateau de table (2) qui est supporté de manière à pouvoir être déplacé dans le sens longitudinal par une pièce de guidage (3) qui est montée à pivot autour d'un axe de pivotement horizontal (16) dans un socle (4), un mouvement de pivotement de la pièce de guidage (3) pouvant être converti au moyen d'une bielle (9) articulée au cadre (1) en un déplacement longitudinal, caractérisé en ce qu'un système de leviers comporte un levier à deux bras (11, 12) qui est monté à pivot autour d'un palier (17) permettant un pivotement à une certaine distance de l'axe de pivotement (16) de la pièce de guidage (3), qu'un organe en forme de barre (14) est monté à pivot sur le socle (4) autour d'un axe (15) parallèle à l'axe de pivotement (16) et que les extrémités libres (13, 10) de l'organe en forme de barre (14) ainsi que la bielle (9) sont articulées aux deux bras de levier (11, 12).

2. Appareil à rayons X suivant la revendication 1, caractérisé en ce que le bras de levier (11) relié à la bielle (9) est monté sur le côté de la pièce de guidage (3) opposé au socle (4), que le bras de levier (12) relié à l'organe en forme de barre (14) est monté de l'autre côté de la pièce de guidage (3) et que les deux bras de levier (11, 12) sont reliés rigidement l'un à l'autre à travers la pièce de guidage (3).

3. Appareil à rayons X suivant l'une quelconque des revendications précédentes, caractérisé par un agencement tel que dans la zone de la position verticale (position pieds en bas) de l'appareil, l'articulation (13) entre l'organe en forme de barre (14) et le premier bras de levier (12) décrit un arc de cercle qui coupe la droite reliant l'axe de pivotement (16) et le point auquel l'organe en forme de barre (14) est articulé au socle (4).

4. Appareil à rayons X suivant l'une quelconque des revendications précédentes, caractérisé en ce que dans la position pieds en bas, l'angle formé entre la bielle et le bras de levier (11) relié à cette bielle est aussi aigu que possible.

Fig.1

Fig.2a

Fig.2b

Fig.2 c

2